# EUROPEAN PATENT SPECIFICATION

(11) **EP 1 421 923 B1**
(45) Date of publication and mention of the grant of the patent: **25.10.2006**
(21) Application number: 02258057.5
(22) Date of filing: 22.11.2002
(51) Int. Cl.: A61F 5/00

(54) **Anal prolapse prevention aid**
Vorrichtung zur Verhinderung des Analprolaps
Dispositif de prevention du prolapsus anal

(43) Date of publication of application: 26.05.2004
(73) Proprietor: Kyoto Kogyo Co., Ltd., Kita Adati-gun, Saitama-ken 362-0803 (JP); Kato, Tomoko, Kita-ku Tokyo 115-0052 (JP)
(72) Inventor: Kusano, Arata, Saitama-ken 362-0803 (JP)
(74) Representative: Paget, Hugh Charles Edward

(56) References cited:
- DE-C- 77 646
- DE-U- 8 614 360
- NL-C- 87 287
- US-A- 1 737 949
- US-A- 4 563 182
- US-A- 4 583 542

## Description

The invention relates to an anal prolapse prevention aid for holding the internal hemorrhoids in place, which easily fall out of the anus, and keeping effects of the medical treatment of piles.

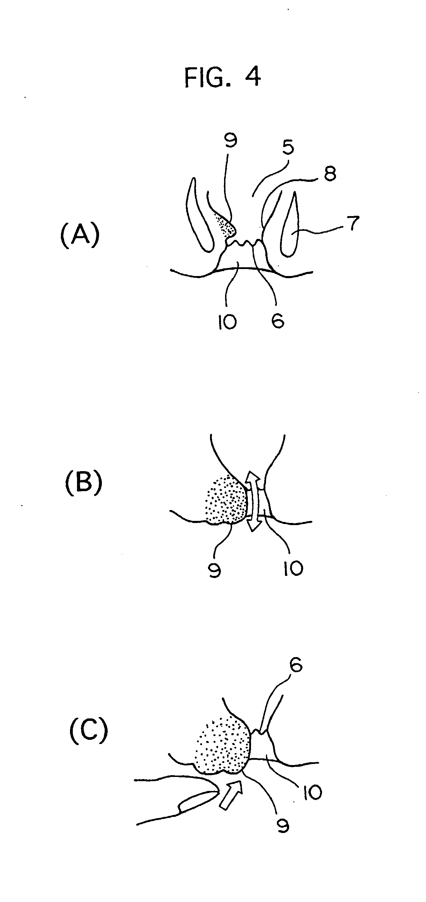

The rectum and the anus at the lower end of the rectum are arranged as illustrated in Fig. 4A. More specifically, the anus 10 lies at the lower end of the rectum 5, with a dentate line extending between the anus 10 and the rectum 5. Toward the rectum 5 the dentate line 6 is associated with so-called cushions 8. These organs are surrounded by an internal sphincter 7 and an external sphincter not shown. In some instances the internal hemorrhoids 9 may project in the region of the cushions 8 as shown in Fig. 4A. The internal hemorrhoids 9 become larger as a result of the cushions 8 themselves swelled up by the congestion arising from various strains which the anus undergoes in daily life and thus from the loosening and breakage of tissues supporting the cushions 8 of the rectum 5.

As the internal hemorrhoids 9 become larger increasingly, the internal hemorrhoids 9 slip out of place upon defecation as shown in Fig. 4, and thereafter the hemorrhoids will naturally return to the inside of the anus. However, when the internal hemorrhoids 9 become further larger and are in the state as illustrated in Fig. 4C, the hemorrhoids remain left outside of the anus, and will not return to the original location unless pushed into place with a finger. Such a state as described above is called an anal prolapse.

Such an anal prolapse is tightened up at its upper portion by the involuntary muscle of the anus. If the internal hemorrhoids 9 remain left outside, such a state will cause the hemorrhoids to become worsen and the trouble to further develop (Cited from "*How to Live with Hemorrhoids Skillfully*", written by Jyun'ichi Iwataru, published by Kodansha).

The conventional anal prolapse prevention aids are known from Japanese Utility Model Application Laid-open (*kokai*) Nos. Hei5-11920 and Sho62-194320 and Japanese Patent Application Laid-open (*kokai*) No. Sho57-318516. These aids are formed of a rubber mold for example, and comprise a head portion inserted into the interior of the anus, a stopper secured to the outside of the anus, and a neck portion located therebetween. Of these portions, the stopper is shaped like the pedestal of a wine glass, crossing the axes of the head portion and the neck portion at right angles. In use, first of all, the anal prolapse is returned to the inside of the anus with a finger, and then, the head of the conventional anal prolapse prevention aid is inserted into the anus. Then, the anus tightens up the neck, and the stopper is located on the outside of the anus.

US 1,737,949 describes a suppository for the treatment of hemorrhoids comprising an inert hollow head or anchoring member and a body in the form of a fabric tube the upper end of which is inserted into the lower end of the head.

As the conventional anal prolapse prevention aid formed from a single-piece molded rubber are stiff, even if the neck and stopper can be deformed to a certain extent, use of the aid always gives the anus a feeling of physical disorder. In addition, since the length of the tightening part of the anus varies depending on the physical constitution or body form of each user, individual differences cannot be accommodated, because when the head is inserted, the stopper comes out of the anus too long, or the head enters the anus too deep.

Furthermore, the stopper projects outward from the anus, and hence it irritates the anus while sitting or walking, and could easily cause pain.

In addition, there is a drawback of not looking nice because the shape of the stopper comes into sight on the so-called panty (underwear)-line, as the stiff stopper comes out to the outer surface of the hip.

Also, while wearing the aid, there is a drawback of difficulty in discharging gases from the rectum.

Therefore, as a result of various experiments and studies, the inventors have developed an ideal anal prolapse prevention aid, which can securely prevent an anal prolapse without giving a feeling of physical disorder to the user.

The invention as defined in claim 1 is an anal prolapse prevention aid comprising a balloon-shaped rubber film with its one end open, the rubber film having a closed end which receives stuffing for the formation of a small-head portion for trapping hemorrhoids in the intestinal wall, the closed end containing the stuffing being closed in the shape of a neck to form a neck portion at its root, the rubber film defining a film portion which extends from the small-head portion via the neck portion, the film portion having at least a length long enough to reach the outside of the anus.

In the invention as defined in claim 2, which depends on claim 1, the stuffing is a sponge a rounded cotton.

In the invention as defined in claim 3, which depends on claim 1, the stuffing is formed from a gas, a liquid or a jelly.

In the invention as defined in claim 4, which depends on any one of claims 1 to 3, the small head portion has a circular or oval cross-section with its maximum diameter being 5mm to 25mm, and the thickness of the film portion is 1mm or less.

In the accompanying drawings:
Fig. 1 is a longitudinal cross-sectional view of an anal prolapse prevention aid according to the invention;
Fig. 2 is an explanatory diagram of a state where an anal prolapse prevention aid according to the invention is inserted in place;
Fig. 3 is an explanatory diagram of a procedure for inserting an anal prolapse prevention aid according to the invention; and
Fig. 4 is an explanatory diagram of the internal hemorrhoids 9 in the rectum 5 and the dentate line 6.

An embodiment of the present invention will now be described with reference to the drawings.

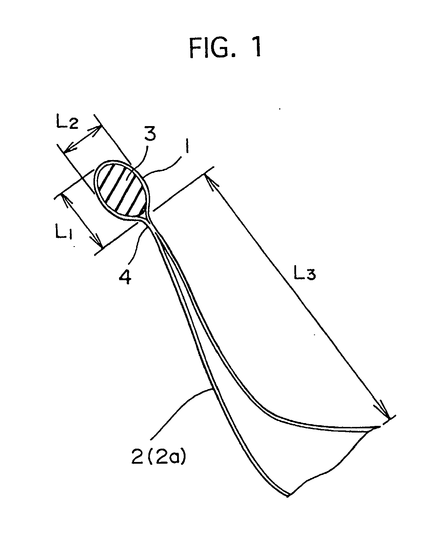

Fig. 1 is a longitudinal cross-sectional view showing the embodiment of an anal prolapse prevention aid according to the invention.

This embodiment comprises a small head 1 with oval cross section, and a film portion 2 continuous with one end of the small head along its longitudinal axis. The small head 1 has a neck 4 which is formed by inserting a stuffing 3 into the closed end of a balloon-shaped rubber film 2a with one end open having a specified or more length so as to allow the closed end including the stuffing 3 to form a neck. To this neck 4, the film portion 2 formed of the rubber film 2a extends on the opposite side of the small head 1.

Preferably, the thickness of the rubber film 2a is 1mm or less. This rubber film has a flexibility but no stiffness at all. The thickness can be, for an example, the thickness of a surgical glove at the thickest, or the thickness of a condom at the thinnest.

The material of the stuffing 3 can be a closed cell foam-based sponge or rounded cotton, or, water or other liquids, or gases such as air or oxygen, or, a jelly form. Preferably, the end of the rubber balloon is fused, bonded or tightened up so that the neck portion 4 is of an airtight or liquid-tight structure, if the gas, liquid or jelly form is used as the stuffing.

### Method of Use

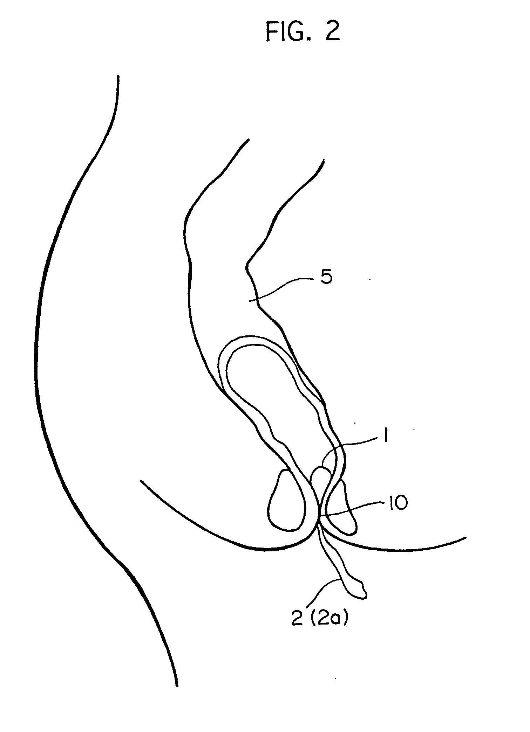

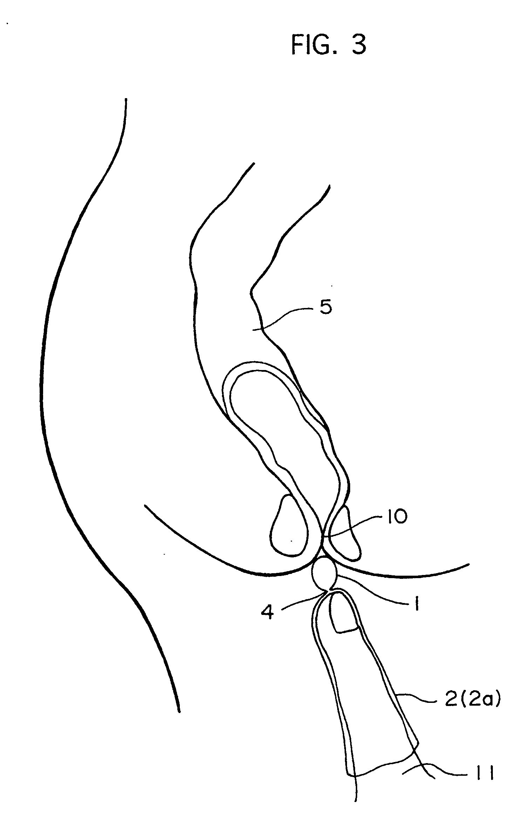

First, the hemorrhoids, which have slipped down, are inserted into the inside of the anus with a finger, and at the same time, a medicine for hemorrhoids is applied to the inside. Next, as shown in Fig. 3, a finger 11 is inserted into the rubber film 2a of the anal prolapse prevention aid according to the invention, and the finger 11 is inserted into the anus 10 entirely, while the rubber film 2a is held with the leading end of the rubber film pushed against the neck 4 as far as it will go. Then, the small head portion 1 is placed deep in the inside of the anus 10 as shown in Fig. 2. The film portion 2 is held and caught in the anus 10, thus the film portion 2 will not return to the outside by its friction resistance. Experiments show that the small head 1 of the aid according to the invention will not come out to the outside, even while walking, sitting or playing sports, and by this feature, hemorrhoids are held inside the anus. This is by the function of the small head 1 for trapping hemorrhoids in the intestinal wall to prevent them from slipping down.

With the rise in the pressure of gas, the gas produced in the intestine is easily discharged from the clearance between the anus 10 and the film portion 2. At this time, according to the experiments, the small head 1 never prolapsed out of the anus 10 to the outside.

Next, while defecating, by means of pulling the leading end of the film portion 2, the anal prolapse prevention aid according to the invention can be easily pulled out of the anus.

In the above-described embodiment, the small head 1 is designed so that in the cross-section, the longer diameter L1 is on the order of 15mm, the shorter diameter L2 is on the order of 12mm, and L3 is on the order of 50-100 mm.

Naturally, the film portion 2 is not limited to the above-mentioned embodiment, and its shape may be like a cord or band. The requirements are that it has flexibility but no stiffness, and does not produce a feeling of physical disorder when the anus tightens up the instrument. Preferably, the length of the film portion 2 when the small head 1 is inserted into the anus is on the order of the length including the length for the anus to tighten up, and additional length extending there from to the outside, in short, the length is long enough to be picked up. Furthermore, the largest diameter of the small head is preferably on the order of 5-25mm, and the thickness of the film portion 2 is preferably 1 mm or thinner, and is as thin as possible, so that it does not give a feeling of physical disorder.

The anal prolapse prevention aid according to the invention can effectively prevent hemorrhoids from prolapsing out of the anus, wherein the film portion 2 having flexibility but no stiffness and continuous with the small head portion 1 is tightened up by the involuntary muscle of the anus when the small head portion 1 is inserted in the anus, and whereby the small head portion 1 itself is prevented from prolapsing to the outside.

Moreover, as the film portion 2 to be tightened by the anus is formed with a flexible material having no stiffness, the anus does not feel physical disorder. At the same time, an excessive part of the film portion 2 coming out to the outside of the anus gets to fit the material of the underwear, and will not deform the so-called panty line.

As the film portion 2 to be tightened by the anus has flexibility but no stiffness, it can discharge the gas in the rectum to the outside while the aid according to the invention is installed. For this reason, it can be an aid, which will not impair the health of users.

Also, as the film portion 2 has more than enough length to reach the outside of the anus, the anal prolapse prevention aid according to the invention can be easily taken out to the outside, by means of pulling the excessive film on the outside of the anus.

Since an embodiment according to the invention as defined in claim 2 comprises a balloon-shaped rubber film 2a with one end open and having a specified or longer length, and a neck portion 4, formed by means of inserting a stuffing 3 for forming a small head 1 into the closed end of the rubber film, and blocking the closed end including the stuffing in a neck-state, the manufacture of such aids is easy, and the surface of the small head portion 1 and the film portion 2 can be formed as a single piece, so that a substantial anal prolapse prevention aid can be provided which gives a satisfactory capability of being inserted into the anus.

As the rubber film 2a with one end open is a balloon-shaped having a specified or longer length, the small head 1 can be easily inserted into the anus, by means of inserting a finger from the open end into the inside, and further inserting the finger into the anus as the leading end opposed against the small head 1, thus this method for inserting is hygienic, and keeps the finger clean.

According to the invention as defined in claim 2, as the stuffing 3 is formed of a sponge body or rounded cotton in claim 1, the stuffing can be easily deformed when the aid according to the invention is inserted or removed, thus such an anal prolapse prevention aid can be provided that does not cause pain.

In the invention as defined in claim 4, as the stuffing 3 is formed from a gas, liquid or a jelly-state substance, the aid according to the invention can be inserted into the anus much more easier. In short, the aid can be inserted or removed easily, as the aid gets fit the anus while inserting or removing.

According to the invention as defined in claim 4, the anal prolapse prevention aid can be inserted into/removed from the anus without giving pain upon insertion and removal.

## Claims

1. An anal prolapse prevention aid comprising a balloon-shaped rubber film (2a) with its one end open, the rubber film (2a) having a closed end which receives stuffing (3) for the formation of a small-head portion (1) for trapping hemorrhoids in the intestinal wall, the closed end containing the stuffing (3) being closed in the shape of a neck to form a neck portion (4) at its root, the rubber film (2a) defining a film portion (2) which extends from the small-head portion (1) via the neck portion (4), the film portion having a least a length long enough to reach the outside of the anus.

2. An anal prolapse prevention aid according to claim 1, wherein the stuffing (3) is a sponge a rounded cotton.

3. An anal prolapse prevention aid according to claim 1, wherein the stuffing (3) is formed from a gas, a liquid or a jelly.

4. An anal prolapse prevention aid according to any one of claims 1 to 3, wherein the small head portion (1) has a circular or oval cross-section with its maximum diameter being 5mm to 25mm, and wherein the thickness of the film portion (2) is 1mm or less.

## Patentansprüche

1. Analprolaps-Vorbeugungshilfsmittel, umfassend eine ballonförmige Kautschukfolie (2a), deren eines Ende offen ist, wobei die Kautschukfolie (2a) ein geschlossenes Ende aufweist, das Füllmaterial (3) aufnimmt, um einen kleinen Kopfabschnitt (1) zur Fixierung von Hämorrhoiden an der Darmwand zu bilden, wobei das geschlossene Ende, welches das Füllmaterial (3) enthält, in Form einer Verengung verschlossen ist, um einen Verengungsabschnitt (4) an seiner Wurzel zu bilden, wobei die Kautschukfolie (2a) einen Folienabschnitt (2) definiert, der sich vom kleinen Kopfabschnitt (1) aus über den Verengungsabschnitt (4) hinaus erstreckt, wobei der Folienabschnitt zumindest lang genug ist, um aus dem Anus herauszuragen.

2. Analprolaps-Vorbeugungshilfsmittel nach Anspruch 1, worin das Füllmaterial (3) ein Schwamm oder ein Baumwollkügelchen ist.

3. Analprolaps-Vorbeugungshilfsmittel nach Anspruch 1, worin das Füllmaterial (3) aus einem Gas, einer Flüssigkeit oder einem Gel besteht.

4. Analprolaps-Vorbeugungshilfsmittel nach einem der Ansprüche 1 bis 3, worin der kleine Kopfabschnitt (1) einen kreisförmigen oder ovalen Querschnitt mit einem maximalen Durchmesser von 5 mm bis 25 mm aufweist, und worin die Dicke des Folienabschnitts (2) 1 mm oder weniger beträgt.

## Revendications

1. Dispositif de prévention du prolapsus anal comprenant un film en caoutchouc en forme de ballonnet (2a) dont une extrémité est ouverte, le film en caoutchouc (2a) ayant une extrémité fermée qui reçoit une garniture (3) pour la formation d'une portion de petite tête (1) pour renfermer les hémorroïdes dans la paroi intestinale, l'extrémité fermée contenant le rembourrage (3) étant fermée sous la forme d'un col pour former une portion de col (4) à sa racine, le film en caoutchouc (2a) définissant une portion de film (2) qui s'étend de la portion de petite tête (1) par la portion de col (4), la portion de film ayant au moins une longueur suffisamment longue pour atteindre l'extérieur de l'anus.

2. Dispositif de prévention du prolapsus anal selon la revendication 1, où la garniture (3) est une éponge entourée de coton.

3. Dispositif de prévention du prolapsus anal selon la revendication 1, où la garniture (3) est constituée d'un gaz, d'un liquide ou d'une gelée.

4. Dispositif de prévention du prolapsus anal selon l'une des revendications 1 à 3, où la portion de petite tête (1) présente une section transversale circulaire ou ovale, son diamètre maximal étant de 5mm à 25mm, et où l'épaisseur de la portion de film (2) est de 1mm au moins.
